Europäisches Patentamt

European Patent Office

Office européen des brevets

(11) Publication number: **0 077 569**
A2

# (12) EUROPEAN PATENT APPLICATION

(21) Application number: **82109650.0**

(22) Date of filing: **19.10.82**

(51) Int. Cl.³: **C 12 N 15/00**
C 12 N 1/20, C 12 N 9/86
//C12R1/19

(30) Priority: **20.10.81 JP 166367/81**

(43) Date of publication of application:
**27.04.83 Bulletin 83/17**

(84) Designated Contracting States:
**BE DE FR GB**

(71) Applicant: **Tamura, Gakuzo**
**2-17-12 Sanno Ohta-ku**
**Tokyo(JP)**

(72) Inventor: **Tamura, Gakuzo**
**2-17-12, Sanno Ohta-ku**
**Tokyo(JP)**

(72) Inventor: **Yoda, Koji**
**5-22-15, Mejiro Toshima-ku**
**Tokyo(JP)**

(72) Inventor: **Kikuchi, Yasuhiro**
**1-8-15, Shoan Suginami-ku**
**Tokyo(JP)**

(72) Inventor: **Yamasaki, Makari**
**4-18,11, Midori-machi Koganei-shi**
**Tokyo(JP)**

(74) Representative: **Casalonga, Axel et al,**
**BUREAU D.A. CASALONGA OFFICE JOSSE & PETIT**
**Baaderstrasse 12-14**
**D-8000 München 5(DE)**

(54) DNA gene.

(57) The invention relates to a DNA gene derived from alkaline phosphatase of *Escherichia coli* and having at least one of the promoter sequence, the shine-dalgarno sequence and the signal sequence.

EP 0 077 569 A2

## TITLE OF THE INVENTION

### DNA GENE

Background of the Invention

The present invention relates to the isolation and utilization of a gene responsible for the formation and secretion of alkaline phosphatase (referred to as APase hereinafter) of Escherichia coli. More specifically, the present invention relates to the isolation and utilization of a DNA gene which is derived from APase gene (referred to as pho A hereinafter) of Escherichia coli, responsible for formation and secretion of proteins and having at least one of the promoter sequence, the Shine-Dalgarno sequence (SD sequence), the signal sequence and a combination thereof.

In order to accumulate abundantly desired products derived from genes in Escherichia coli, such improvements as increase of the gene dosage in cells and increase of the rate of transcription and translation of the genes have been developed. Increase of gene dosages is carried out by using as a vector a Col El plasmid, a copy-number mutant derived from R factor plasmid, or a plasmid containing phage replication system.

For the expression of foreign genes, such efficient promoters as E. coli lactose operon promoter, E. coli tryptophane operon promoter, λphage promoters and the like are employed. The length between promoters (including SD) and initiation codon of the desired genes is adjusted to maximize the expression of the genes. It has been reported that when a cloned E. coli trp operon on a multi-copy plasmid was induced, 55% of the proteins synthesized de novo and 30% of the total protein are the trp ED gene product. It is expected that the use of the trp promoter will result

- 2 -                              **0077569**

in the production of considerable amount of desired proteins.

However, these methods have not necessarily led to the production of large amounts of foreign proteins as expected. This is probably because, in many cases, the production of proteins is restricted by the harmful effects of the production against the host bacteria such as consumption of particular tRNA species in E. coli due to the difference in codon preference by foreign genes and disorders in the metabolism in host bacteria by the activity of the products, and by rapid decomposition of the protein recognized as foreign products.

Most of these problems will be solved by causing the gene products to be secreted outside of the cytoplasma, i.e. in the periplasmic space or in the medium. In this way, it becomes possible to produce harmful products for cell metabolism and also to protect gene products from degradation. It also becomes possible to produce in large amounts by releasing from feed back inhibition a gene product which inhibits its own synthesis by itself or its derivatives when produced in large excess. Further, in the process of fermentation, it is considered easier or more efficient to recover and purify gene products (proteins) from culture media than from cells. When a Gram positive bacterium such as Bacillus subtilis is used as a host microorganism, proteins can be accumulated outside the cell membrane, and therefore, can be recovered from the medium. In case of a Gram negative bacterium such as Escherichia coli, proteins accumulated in the periplasma can be selectively extracted by osmotic shock, and in case of periplasm-leaky mutants, proteins may be excreted in the medium.

The mechanism for the secretion of proteins from cells is explained by Blobel's signal hypothesis [Blobel G. & B. Dobberstein, J. Cell Biol., 67, 835 (1975)] as follows:

1.    In the biosynthetic process, secreted proteins are synthesized as a precursor which has at the amino-terminal a signal peptide of 15 - 30 amino acid residues rich in hydrophobic amino acids.

2.    The signal peptide binds the protein synthesizing system initiated by the same mechanism as the synthesis of cytoplasmic proteins to the cytoplasmic membrane through the interaction with the receptor on the membrane and induces the formation of pores in the membrane to achieve translocation of polypeptide coupled with its synthesis.

3.    The signal peptide is cleaved off by a protease in the membrane during polypeptide synthesis.

This hypothesis presented as an explanation for the mechanism of the synthesis and secretion of secreted proteins in animal cells has been gradually accepted because the studies of the structure of messsenger RNAs or DNAs of secreted proteins and membrane proteins in many other animals and microorganisms have made it clear that most of these secreted proteins have the amino-terminal signal peptides cleaved off to make mature proteins.  Moreover, the analysis of Escherichia coli mutant strains proved that the signal peptide region has an important role in the early stage of secretion because mutants with low level of secretion were found to have substitutions of hydrophobic amino acids in the signal sequence with acidic or basic amino acids.

Brief Description of the Drawings

Fig. 1 shows the process for constructing the DNA gene of the present invention.  In Fig. 1, E, H, X, B, S and f represent the restriction sites for EcoRI, HindIII, XhoI, BamHI, SmaI and HinfI, respectively.

Fig. 2 shows the nucleotide sequence of the DNA gene of the present invention.  In Fig. 2, HindIII, AluI, HhaI, BclI, MboI, HaeII, PvuII, XmaIII, HaeIII, HpaII, HinfI and BamHI represent the cleavage sites for the respective restriction endonucleases, PB represents Pribnow box sequence, SD represents Shine-Dalgarno sequence, and iso-1 and iso-3 represent iso-enzymes 1 and 3, respectively.  In Fig. 2, A, T, G and C represent adenine, thymine, guanine and cytocine, respectively.

Fig. 3 shows the process for constructing pYK225. In Fig. 3, bla represents ß-lactamase gene and Kan represents the gene responsible for kanamycin-resistance.  The other symbols have the same meanings as defined above.

Fig. 4 shows electrophoresis proving the presence of phoA' - 'bla fused protein induced under regulation of phosphate.  In Fig. 4, + represents the culture with 1 mM phosphate and - represents the culture without phosphate. m represents marker-protein for molecular weight and K is kilodalton.

Fig. 5 shows electrophoresis illustrating the change with time in the amount of phoA' - 'bla fused protein produced by YK 811 under low concentration of phosphate.

Fig. 6 shows electrophoresis proving the presence of the protein produced in the periplasmic space under low concentration of phosphate.  In Fig. 6, A, B, C, D, h and p represent pYK 227, pYK 228, pYK 229, pYK 230, host microorganism Escherichia coli MC 1061 and pYK 225, respectively.

Description of the Invention

The present inventors have studied the gene encoding APase, one of the periplasmic enzymes of Escherichia coli [L. Heppel, "Structure and Function of Biological Membranes 223, (1971)].  The enzyme is a dimer comprising the monomers each having a molecular weight of 43,000 and 2 atoms of Zn and synthesized inducibly in an inorganic phosphate-deficient medium in a high yield of 6% of the proteins synthesized de novo.  Accordingly, a desired polypeptide can be produced and secreted into the periplasm in a high yield under the control of phosphate concentration in a medium by cloning a DNA segment containing pho A promoter and signal sequence and inserting a gene encoding the desired peptide downstream to the signal sequence.

The present inventors have cloned a DNA fragment containing the amino-terminal and 5'-flanking region of APase including a promoter by using a promoter cloning vector pMC 1403 [M. Casadaban et al., J. Bacteriol. 143, 971 - 980, 1980] and determined the nucleotide sequence of the cloned fragment. As a result, it has become possible to insert a gene encoding a desired peptide downstream to the pho A promoter to enable efficient expression of the gene. Further, a desired gene may be inserted downstream to the signal sequence of APase in a proper reading frame so that it can be expressed and secreted. Thus, the present invention providing a method for production and secretion of desired peptides has been completed.

Isolation of the DNA gene of the present invention and determination of the nucleotide sequence thereof are carried out as follows.

(1) Preparation of pho A-transducing phage

A strain of Escherichia coli lacking λ attachment site, for example, Escherichia coli K-12 KS 302 [Molec. Gen. Genet., 137, 101 (1975)] is infected with a phage, for example, λb515 b519 xis 6 cI 857 S7 nin 5 phage (refer to the reference mentioned above) and a pho A-transducing phage λdpho-1 is obtained by the mixed lyzate method (refer to the reference mentioned above) using pho A deficient-Escherichia coli YK 514 (F⁻, thi leu lac Y gal K rps Lpho A8 rk⁻ mk⁻) as a recipient cell.

(2) Cloning of the DNA fragment containing pho A and expression-controlling site thereof to pBR322

λdpho-1 DNA and plasmid pBR322 are double-digested with the same restriction enzyme such as HindIII and BamHI, respectively, and both digests are mixed. The mixture is treated with T4 ligase to obtain a plasmid pKI-2 to which pho A is cloned.

(3) Cloning of pho A promoter region

pKI-2 and a promoter cloning vector DNA such as pMC 1403 [J. Bacteriol., 143, 971 - 981 (1980)] are mixed and digested with a restriction enzyme such as EcoRI. The digest is treated with T4 ligase to obtain plasmid pYK 171 containing pho A promoter region. pYK 171 is partially digested with a restriction enzyme such as HinfI and then treated with a restriction enzyme such as SmaI to obtain plasmid pYK 190 which is shorter than pYK 171 but contains pho A promoter region.

(4) pYK 190 is double-digested with restriction enzymes such as HindIII and BamHI and a fragment of 520bp containing pho A promoter is isolated. The nucleotide sequence of the fragment is determined by Maxam & Gilbert method [A. M. Maxam and W. Gilbert, Methods in Enzymology, Academic Press, LXV, 499 - 560 (1979)].

The determination of nucleotide sequence and the preparation of restriction map have enabled the utilization of many restriction sites as cloning sites for desired genes.

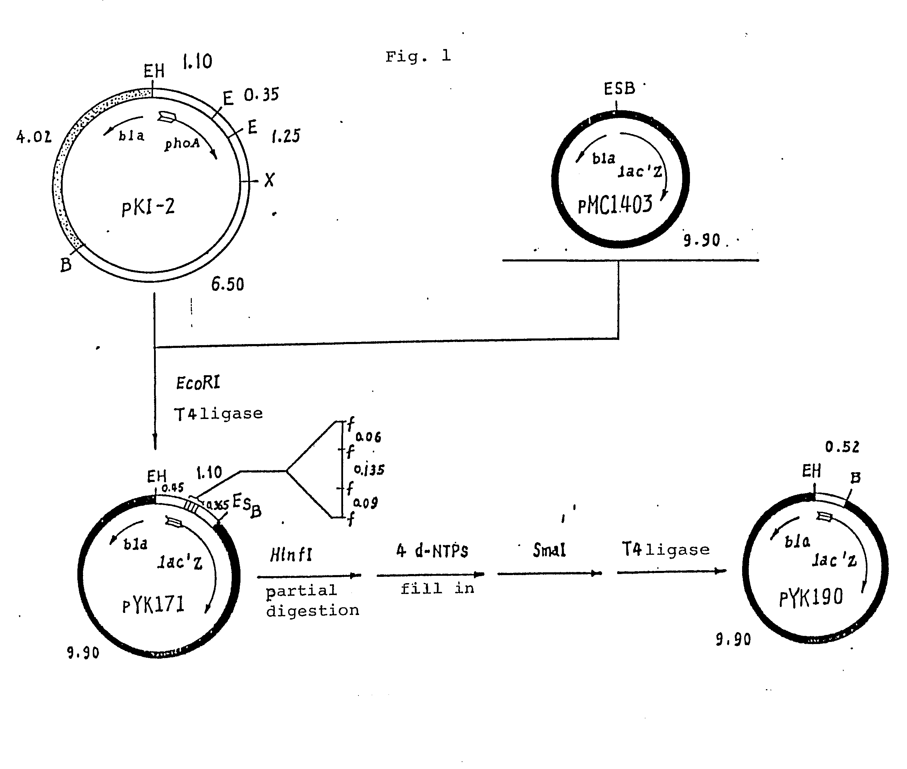

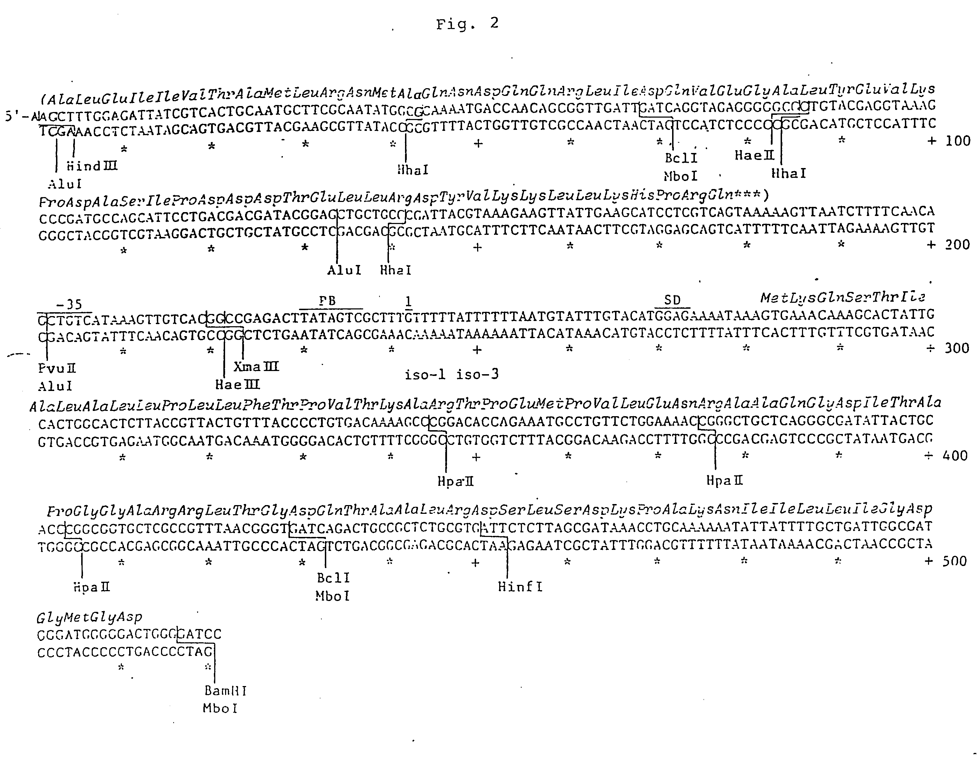

(1) As illustrated in the sequence of Fig. 2, the HpaII site between the triplet corresponding to alanine which is the last amino acid of pho A signal sequence and that corresponding to arginine which is the amino-terminal amino acid of its subunit iso-1 APase (an isozyme of APase) two HpaII sites downstream thereto, the BclI site, the MboI site, the HinfI site, the BamHI or MboI site derived from pMC 1403 and two EcoRI sites shown in pKI-2 of Fig. 1 are preferably employed. The use of a cloning site more downstream to the signal sequence will result in the expression of a fused protein having a longer amino-terminal region of APase preceding that of a desired peptide.

(2)　A gene encoding a desired peptide is cut at the restriction site as close as possible to the amino-terminal of the desired peptide and ligated to pho A promoter. Most preferably, the promoter region is ligated directly to the first nucleotide of the triplet of a desired peptide.

(3)　The ligation of the two fragments, i.e. the fragment containing pho A promoter sequence, SD sequence and signal sequence and another fragment encoding a desired gene may be carried out on a plasmid or before the insertion into a plasmid. Since the nucleotide sequence of any DNA fragment can be determined, especially near the end of a DNA fragment, the two fragments can be ligated in a proper reading frame according to various situations. Even if the nucleotide sequence of a desired gene is not known, it is still possible to obtain a clone having a proper reading frame by the selection with the expression of the desired phenotype among transformants after transformation with ligated mixture of the promoter and a desired gene digested with a nuclease such as BAL31.

(4)　When a desired gene can not be cut near the amino-terminal of the peptide or it is desired to produce the desired peptide which neither has excess amino acids at the amino-terminal nor lacks inherent amino acids, a nucleotide sequence which corresponds to the sequence upstream from the first internal restriction site available is synthesized and used to fill the gap between the signal sequence and the first internal restriction site of a desired gene, so that an intact desired peptide can be produced.

The present invention is explained more in detail by the following examples.

Example 1

Construction of plasmid pYK 190 containing pho A promoter, SD sequence and signal sequence:

(1)  Preparation of pho A transducing phage:

Pho A, the structural gene for APase is present at 8.7 minutes on E. coli genetic map.  In order to concentrate the desired gene as the first step for cloning pho A, a special transducing phage was prepared in the following manner according to the mixed lysate method [W. J. Schrenk et al., Molec. Gen. Genet., 137, 101 (1975)]. The mixed lysate method is a modification of the secondary attachment site method for shot gun selection which was developed by Shimada et al to obtain transducing phages. The selection was done as follows.

Escherichia coli K-12 KS 302 (HfrH, thi met Δgal-bio) (refer to the reference mentioned above) lacking the primary attachment site attλ was infected with λb515 b519 xis 6c I 857 S7 nin 5 phage and plated on a bouillon agar medium on which phageλ ch 80 del 9  [Shimada et al., J. Mol. Biol., 63, 483-503 (1972)] (which can infect λ-phage resistant strains and kill non-lysogenic strains but itself can not lysogenize), followed by incubation at 30°C for 30 hours to form the colonies of the lysogenic strain. In this case, since attλ is lost, λphages in the lysogenic strains were integrated into the chromosome at random at sites with homologous nucleotide sequences to attP.P'.  About $10^4$ colonies were combined, cultivated and subjected to induction by heating at 42°C for 15 minutes to obtain a mixed lysate having about 5 x $10^{11}$ plaque forming units (U/ml) and containing 2.5 x $10^8$ p.f.u/ml of λch 80 del 9. The lysate contained transducing phages having a DNA near various secondary attachment sites incorporated into the phage chromosome by illegitimate recombination and thus the transducing phages can introduce various genetic markers. phoA-deleting Escherichia coli YK 514 (F⁻, thi leu lac Y gal K rps L phoA 8 rk⁻ mk⁻) was infected with the lysate at a multiplicity of infection of 10 to 100 and spread on an agar plate prepared by adding 40 mg/ml 5-bromo-4-chloro-3-indolylphosphate-p-toluidine (referred to as XP hereinafter) to a medium 121 consisting of 15 g/l agar, 4 g/l glucose, 4.68 g/l sodium chloride, 1.5 g/l potassium chloride, 1.08 g/l ammonium chloride, 0.35 g/l sodium sulfate, 0.2 g/l magnesium chloride, 0.029 g/l calcium chloride, 0.5 mg/l ferric chloride, 0.27 mg/l zinc chloride and 12 g/l trishydroxymethylaminomethane and adjusted to pH 7.5 with hydrochloric acid. After incubation at 30°C for 2 days, blue pho A$^+$ colonies were obtained with a frequency of several per $10^8$. Escherichia coli YK 514 is a phoA-deleting mutant prepared by the fusion of Escherichia coli 15 [Suzuki & Garen, J. Mol. Biol., 45, 549-566 (1969)] with Escherichia coli RRI [Bolivar et al., Gene 2, 95-113 (1977)].

Then, about 50 selected pho A$^+$ transductants were subjected to heat induction at 42°C for 15 minutes to obtain high frequency-transducing lysates. Plaque-forming phages were not obtained from the lysates.

A large amount of lysate was prepared from a strain with high transducing ability. The lysate was treated with 0.5 M NaCl and 10% polyethyleneglycol and the resulting precipitate was recovered by centrifugation. It was subjected to cesium-chloride density gradient centrifugation and a band of transducing phage (107.3% λDNA) was recognized at a higher density than the helper phage, λb515 b519 xis 6cI 857 S7 nin 5 (90.4% λDNA). The transducing phage was isolated and named λdpho-1.

The DNA in the purified λdpho-1 was extracted by phenol method and a cleavage map thereof was prepared by digestion with combinations of restriction endonucleases BamHI, EcoRI and HindIII (products of Takara Shuzo Co.). As a result, it was found λdpho-1 is a λgal type deletion phage having BamHI site at 47Kb from the right end and a new EcoRI site at 39Kb, it is assumed that λdpho-1 has Escherichia coli DNA of 17-25Kbp. As λdpho-1 can transform

$\lambda$-lysogenic <u>Escherichia coli</u> pho A 8 rec Al [Yoda <u>et al</u>., Agric. Biol. Chem., <u>45</u>, 549-566 (1980)] to pho A$^+$ and the transformants produce APase inducibly under low phosphate concentrations, it is concluded that $\lambda$dpho-l has the entire pho A gene and is expressed by its own promoter.

(2)    Cloning of a DNA fragment containing pho A and its regulator gene to pBR 322:

$\lambda$dpho-l DNA was cut with three restriction endonucleases BamHI, EcoRI and HindIII alone or in combination which were used for making the cleavage map.  The resulting fragments and plasmid pBR322 fragments cut with the same enzymes were ligated with T4 ligase, followed by transformation of <u>Escherichia coli</u> pho A 8 rec A l. Recombinant plasmids in pho A$^+$ Ap$^R$ transformants were checked for their structures.

The smallest of the obtained plasmids is a 13.2Kbp plasmid wherein 9.2Kbp <u>Escherichia coli</u> DNA cut out with HindIII and BamHI was cloned.  This plasmid is named pKI-2 (refer to Fig. 1).  The formation of slightly blue colonies of the transformant on a medium 121 plate containing 1 mM phosphate and XP shows the low level of expression of APase.  The expression results from the derepression owing to the increase in the copy number of pho A gene.  The phenomenon, which is called "titration out" of the repressor, has been observed with lac Z gene.  The remarkable induction of APase activity under regulation of phosphate proves the cloning of entire pho A gene.

The 9.2Kbp fragment in pKI-2 was cut with XhoI (product of Takara Shuzo Co., which cuts C$^{\downarrow}$TCGAG, one of the cleavage sequences of AvaI) to form two fragments, 2.7Kbp and 6.5Kbp.  The plasmid having the 2.7Kbp fragment shows pho A$^+$ transformation activity and the synthesis of APase in the transformant was regulated by the concentration of phosphate.  Therefore, the 2.7Kbp fragment contains entire pho A gene.

(3)    Cloning of pho A promoter region:

Cloning of pho A promoter region from the plasmid pKI-2 containing pho A was carried out as follows.

A mixture of pKI-2 and the promoter cloning vector pMC1403 [refer to J. Bacteriol., 143, 971-980 (1980)] was digested with EcoRI at 37°C for 1 hour and treated with T4 ligase (product of Takara Shuzo Co.) at 15°C for 24 hours to ligate both plasmids. The reaction mixture was used to transform Escherichia coli MC 1061 strain (Δlac Z) [Casadaban et al., J. Bacteriol. 143, 911-980 (1980)] by a conventional method [S. N. Cohen et al., Proc. Natl. Acad. Sci., U.S.A., 69, 2110-2114 (1972)]. Transformants which were ampicillin-resistant and showed Lac$^+$ under low concentration of phosphate were selected on a medium 121 containing 5-bromo, 4-chloro, 3-indolyl-D-galactoside. Plasmid pYK 171 in one of such transformants has four HinfI sites on the 1.10Kbp fragment inserted at EcoRI site (refer to Fig. 1). pYK 171 was partially digested by treating 40 µg of DNA and 1 unit of HinfI (product of Bethesda Research Laboratories) in a buffer solution (pH 7.4) consisting of 7 mM Tris-HCl, 7 mM MgCl$_2$, 7 mM mercaptoethanol and 10 mM NaCl at 37°C for 60 minutes to cut only one HinfI site on pYK 171. The cohesive ends of linearized pYK 171 were filled in with four deoxyribonucleotide-triphosphate and DNA polymerase I (product of New England Biolabs). The reaction was carried out using 125 µM each of deoxyribonucleotide-triphosphate and 0.4 unit of DNA polymerase at 30°C for 60 minutes in 50 µl of 40 mM potassium phosphate buffer (pH 7.5) containing 6 mM MgCl$_2$ and 1 mM 2-mercaptoethanol. 10 µg of the thus obtained DNA was treated with 10 units of SmaI at 37°C for 60 minutes in the same buffer solution as above to construct plasmids having pho A promoter activity and smaller than pYK 171. One of the plasmids, pYK 190 (refer to Fig. 1) contains a fragment of 520 bp between HindIII and BamHI and gives Lac$^+$ transformants under low concentration of phosphate.

The transformant containing pYK 190 has been deposited with Fermentation Research Institute, Agency of Industrial Science and Technology, Chiba, Japan under accession number Escherichia coli YK 734 FERM P-6184.

Example 2

Determination of nucleotide sequence of the 520 bp DNA fragment containing E. coli pho A promoter, SD sequence and signal sequence:

Determination of nucleotide sequence of the 520 bp fragment in pYK 190 was carried out as follows.

pYK 190 was double digested with HindIII and BamHI and a fragment of 520 bp was isolated and purified by polyacrylamide gel electrophoresis [refer to the reference of Maxam and Gilbert mentioned above]. The 5' ends of the fragment were phosphorylated with $[\gamma-^{32}P]$ ATP and the nucleotide sequence was determined by the method of Maxam and Gilbert. The sequence is shown in Fig. 2.

Example 3

Expresssion and secretion of a fused protein containing ß-lactamase using the DNA fragment containing E. coli pho A promoter, SD sequence and signal sequence:

(1) Construction of plasmid pYK 225:

The plasmid pKI-2 was double digested with HindIII - EcoRI to obtain a DNA fragment of 1.1Kbp containing the pho A promoter, SD sequence and signal sequence. That is, 10 ug of the pKI-2 DNA was treated at 37°C for 1 hour with 20 units of HindIII and 20 units of EcoRI. Reaction was carried out in 50 µl of the buffer solution for HindIII [20 mM Tris-HCL (pH 7.4), 7 mM $MgCl_2$, 60 mM NaCl] and the reaction was stopped by heating at 70°C for 5 minutes.

The reaction mixture was subjected to agarose gel electrophoresis to isolate the 1.1Kbp fragment. The

electrophoresis was carried out using 1.0% agarose gel (20 x 15 x 0.5 cm) at 150 volt for 2 hours in 40 mM Tris-acetate buffer (pH 7.8).  Then, the gel was stained with 0.5 µg/ml ethydium bromide for 5 to 10 minutes.  After staining, the desired 1.1Kbp DNA was cut under ultraviolet radiation and put in a pyrex tube (inside diameter: 5 mm) with dialysis membrane at the bottom and extracted by electrophoresis (5 mM / tube in the same buffer for one hour).

The thus obtained 1.1Kbp HindIII - EcoRI fragment and pBR322 treated in the same manner were ligated with T4 ligase (product of Takara Shuzo Co.).  The reaction was carried out at 15°C for 24 hours using 10 µg of pBR322 fragment, 1 µg of the 1.1Kbp fragment and 5 units of T4 ligase in 20 µl of the buffer for ligation [20 mM Tris-HCl (pH 7.6), 10 mM MgCl$_2$, 10 mM dithiothreitol, 0.5 mM ATP].  The thus obtained plasmid was named pKI-4.

Then, pKI-4 was double digested with HindIII and BamHI using the buffer for HindIII.

Separately, Escherichia coli K-12D6434 [Felton et al., J. Bacteriol., 142, 221-228 (1980)] was infected with λcI 857 rex : : Tn 5 Oam 29 Pam 80 b 221 [the same reference as mentioned above) and cultured in a conventional manner to obtain a lysate.  Chloroform was added to the lysate to disrupt cells completely.  The lysate was suspended in a mixture of 10% polyethyleneglycol 6,000 (product of Nakarai Kagaku Co.) and 0.5 M NaCl and incubated at 4°C for 20 hours.  The resulting precipitate was recovered by centrifugation at 10,000 rpm for 10 minutes and a DNA fragment was recovered by the phenol method.

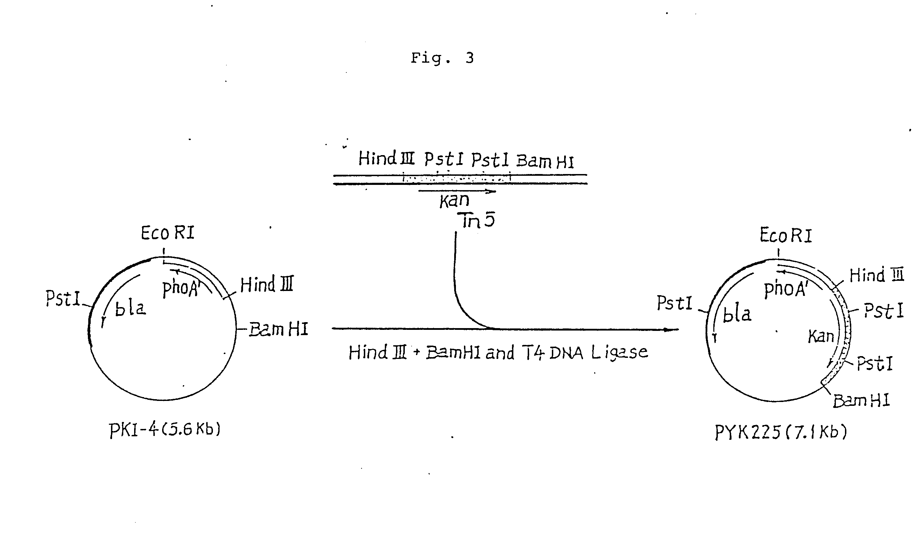

The DNA was double digested with HindIII - BamHI and mixed with pKI-4 digested as above.  The mixture was treated with T4 ligase and Escherichia coli MC 1061 strain was transformed with the reaction mixture, followed by selection of kanamycin-resistant strains on a bouillon medium (product of Eiken Kagaku Co., Ltd.) containing 20 µg/ml kanamycin.  A plasmid in one of the strains was named plasmid pYK 225. The construction of pYK 225 is illustrated in Fig. 3.

(2) Construction of pho A' - 'bla fused plasmid:

In this step, 10 ug of pYK 225 was digested at 37°C for 2 hours with 20 units of EcoRI in 50 µl of the buffer for EcoRI [100 mM Tris-HCl (pH 7.4), 5 mM MgCl$_2$, 50 mM NaCl] and a linearized DNA was recovered as precipitates using ethanol.

Then, about 8 µg of the linearized DNA was treated with 2 units of the exonuclease BAL 31 (product of Bethesda Research Laboratories) in a buffer solution consisting of 20 mM Tris-HCl (pH 8.1), 12 mM MgCl$_2$, 1 mM EDTA and 200 mM NaCl at 30°C for 3 - 7 minutes. The reaction was stopped by heating at 70°C for 5 minutes.

After cooling, the DNA was circularized using T4 ligase and _Escherichia coli_ MC 1061 was transformed with the circularized DNA, followed by selection of kanamycin-resistant strains on bouillon-agar plates (product of Eiken Kagaku Co., Ltd.) containing 20 µg/ml kanamycin. Then, colonies formed on the plate were replica-plated on bouillon media containing 50 µg/ml and 100 µg/ml ampicillin, respectively. Colonies resistant to 50 µg/ml ampicillin and sensitive to 100 µg/ml ampicillin were selected. Among the thus obtained 11 strains, one strain was selected which synthesized a protein larger than β-lactamase (MW 29,000 dalton) in large amounts under the control of phosphate. The strain was cultured in the medium 121 containing 1 mM phosphate. Cells were recovered and cultured again in the medium 121. Cells were recovered at OD 0.7 and disrupted by sonic oscillation. SDS (sodium dodecyl sulfate) was added to a final concentration of 1% and the mixture was heated at 100°C for 50 seconds and then subjected to 12.5% polyacrylamide gel electrophoresis. The proteins in the gel were stained with Coomassie Brilliant Blue (product of Nakarai Kagaku Co., Ltd.). Four strains showed a strong band which seemed to be fused proteins with β-lactamase.

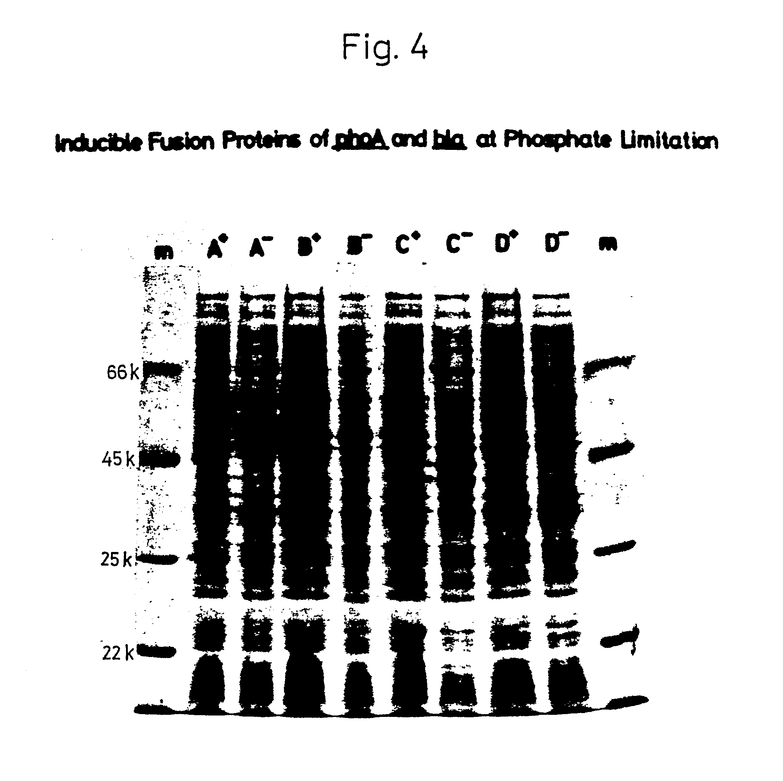

In order to prove that the synthesis of these proteins are dependent on the pho A promoter, the protein bands produced with or without 1 mM phosphate were compared (refer to Fig. 4).

As the result, it was found that the microorganisms containing plasmid pYK 227 (A), pYK 228 (B), pYK 229 (C) and pYK 230 (D) produced 55 kilodalton (referred to K hereinafter), 51K, 41K and 35K proteins, respectively, without addition of phosphate. Escherichia coli YK 811 strain containing plasmid pYK 227 and Escherichia coli YK 814 strain containing plasmid pYK 230 have been deposited with Fermentation Research Institute, Agency of Industrial Science and Technology, Chiba, Japan under accession numbers FERM P-6185 and 6186, respectively.

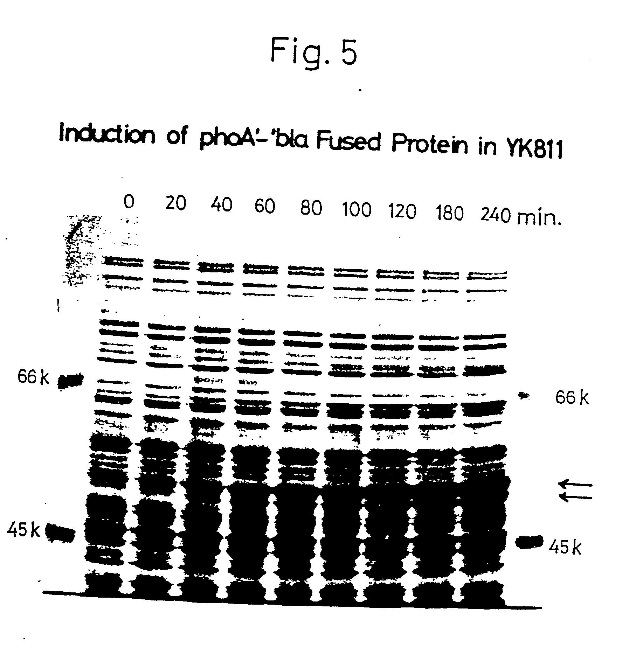

In order to prove that the production of the fused protein depends on the pho A promoter, YK 811 strain containing pYK 227 was cultured in the medium 121 without added phosphate and syntheses of proteins were followed and it was confirmed that the proteins in two bands increased with time (refer to Fig. 5).

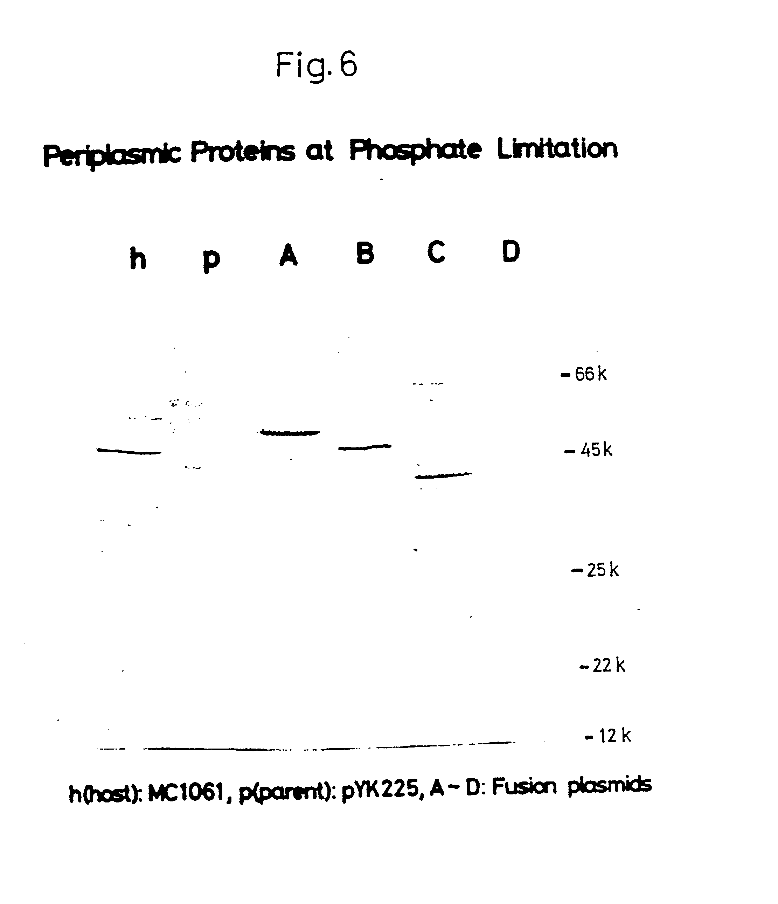

Then, the secretion of these fused proteins to the periplasmic space was examined. That is, each strain was cultured in the medium 121 and subjected to osmotic shock [L. Heppel, Structure and Function of Biological Membranes p. 223 (1971)]. The shocked fluid was analyzed by SDS-polyacrylamide gel electrophoresis, whereby it was proved that the microorganisms having pYK 227, pYK 228, pYK 229 and pYK 230 secreted the fused proteins of 55K, 51K, 41K and 35K, respectively, to the periplasmic space (refer to Fig. 6).

The extent of BAL 31 digestion was estimated by digesting each plasmid with HincII which cuts the plasmid on both sides of the EcoRI site used for BAL 31 digestion. That is, the sizes of DNA fragments formed by treatment with HincII (product of Takara Shuzo Co.) in a 7 mM Tris-HCl (pH 7.4) buffer solution containing 7 mg of $MgCl_2$ and 7 mM 2-mercaptoethanol at 37°C for 1 hour were measured by agarose gel electrophoresis in a conventional manner. As a result, it was found that pYK 227, pYK 228, pYK 229 and pYK 230 lost 460bp, 540bp, 840bp and 880bp, respectively, and especially pYK 229 and pYK 230 lost completely the signal peptides of ß-lactamase [refer to J.G. Sutcliffe, Proc. Natl. Acad. Sci., 75, 3737-3741 (1978)]. Therefore, it is considered that the secretion of the fused proteins to the periplasmic space at least by pYK 229 and pYK 230 as well as the biosynthesis thereof depends on pho A.

WHAT IS CLAIMED IS:

1.     A DNA gene derived from alkaline phosphatase gene (pho A) of Escherichia coli and having at least one of the promoter sequence, the Shine-Dalgarno sequence (SD sequence) and the signal sequence.

2.     The DNA gene according to claim 1, wherein the sequences are those illustrated in Fig. 2.

3.     A strain of Escherichia coli which has a recombinant DNA containing a DNA gene claimed in claim 1.

4.     A recombinant DNA which has a DNA gene claimed in claim 1 and a foreign DNA coding for a desirable protein.

5.     The recombinant DNA according to claim 4, wherein the desirable protein is β-lactamase.

6.     A strain of Escherichia coli which has a recombinant DNA claimed in claim 4.

Fig. 1

Fig. 2

```
(AlaLeuGluIleIleValThrAlaMetLeuArgAsnMetAlaGlnAsnAspGlnGlnArgLeuIleAspGlnValGluGlyAlaLeuTyrGluValLys
5'-AAGCTTTGGAGATTATCGTCACTGCAATGCTTCGCAATATGGCGCAAAATGACCAACAGCCGGTTGATTGATCACGTAGAGGGCGCCGTGTACGACGTAAAG
   TCGAAACCTCTAATAGCAGTGACGTTACGAAGCGTTATACCGCGTTTTACTGGTTGTCGGCCAACTAACTAGTCCATCTCCCGCGGCGACATGCTCCATTTC  + 100
       *              *        *        *     *         +           *           *         *     *
     |                                                                                BclI    HaeII
     HindIII                                                                          MboI    HhaI
     AluI                                         HhaI
ProAspAlaSerIleProAspAspAspThrGluLeuLeuArgAspTyrValLysLysLeuLeuLysHisProArgGln***)
CCCGATGCCAGCATTCCTGACGACGATACGGAGCTGCTGCCCGATTACGTAAAGAAGTTATTGAAGCATCCTCGTCAGTAAAAAGTTAATCTTTTCAACA
GGGCTACGGTCGTAAGGACTGCTGCTATGCCTCGACGACGGCGCTAATGCATTTCTTCAATAACTTCGTAGGAGCAGTCATTTTTCAATTAGAAAAGTTGT  + 200
     *         *        *       *     *        +        *       *        *       *       *
                                  AluI  HhaI

-35                             PB          1                              SD        MetLysGlnSerThrIle
CCTGTCATAAAGTTGTCACGGCCCGAGACTTATAGTCGCTTTGTTTTTATTTTTTAATGTATTTGTACATGGAGAAAATAAAGTGAAACAAAGCACTATTG
GGACAGTATTTCAACAGTGCCGGGCTCTGAATATCAGCGAAACAAAAATAAAAAATTACATAAACATGTACCTCTTTTATTTCACTTTGTTTCGTGATAAC  ÷ 300
     *         *        *       *     *        +        *       *        *       *       *
     PvuII            XmaIII
     AluI             HaeIII                      iso-1 iso-3

AlaLeuAlaLeuLeuProLeuLeuPheThrProValThrLysAlaArgThrProGluMetProValLeuGluAsnArgAlaAlaGlnGlyAspIleThrAla
CACTGGCACTCTTACCGTTACTGTTTACCCCTGTGACAAAAGCCCGGACACCAGAAATGCCTGTTCTGGAAAACCGGGCTGCTCAGGGCCGATATTACTGC
GTGACCGTGAGAATGGCAATGACAAATGGGGACACTGTTTTCGGGCCTGTGGTCTTTACGGACAAGACCTTTTGGCCCGACGAGTCCCGCTATAATGACG  ÷ 400
     *         *        *       *     *        +        *       *        *       *       *
                                       HpaII                                  HpaII

ProGlyGlyAlaArgArgLeuThrGlyAspGlnThrAlaAlaLeuArgAspSerLeuSerAspLysProAlaLysAsnIleIleLeuLeuIleGlyAsp
ACCCGCCGGTGCTCGCCGTTTAACGGGTGATCAGACTGCCGCTCTGCGTGATTCTCTTAGCGATAAACCTGCAAAAAATATTATTTTGCTGATTGGCCAT
TGGGCGGCCACGAGCGGCAAATTGCCCACTAGTCTGACGGCGAGACGCACTAAGAGAATCGCTATTTGGACGTTTTTTATAATAAAACGACTAACCGGCTA  + 500
     *         *        *       *     *        +        *       *        *       *       *
     HpaII                        BclI                  HinfI
                                  MboI

GlyMetGlyAsp
CGGATGGGGGACTGGCCATCC
CCCTACCCCCTGACCCCTAG
     *       *
             BamHI
             MboI
```

Fig. 3

PKI-4(5.6 Kb)

Hind III  PstI  PstI  BamHI

Kan

Tn 5

Hind III + BamHI and T4 DNA Ligase

PYK225(7.1Kb)

9/2

0077569

# Fig. 4

Inducible Fusion Proteins of phoA and bla at Phosphate Limitation

# Fig. 5

## Induction of phoA'-'bla Fused Protein in YK811

# Fig. 6

## Periplasmic Proteins at Phosphate Limitation

h   p   A   B   C   D

— 66k

— 45k

— 25k

— 22k

— 12k

h(host): MC1061, p(parent): pYK225, A ~ D: Fusion plasmids